# EUROPEAN PATENT SPECIFICATION

(11) **EP 2 432 891 B1**
(45) Date of publication and mention of the grant of the patent: **04.04.2018**
(21) Application number: 09844959.8
(22) Date of filing: 19.05.2009
(51) Int. Cl.: A61K 36/258, A23L 29/00, A23L 27/10, A23L 33/105

(54) **METHODS FOR PREPARING A FERMENTED GINSENG CONCENTRATE OR POWDER**
VERFAHREN ZUR HERSTELLUNG EINES FERMENTIERTEN GINSENGKONZENTRATS ODER -PULVERS
PROCÉDÉS DE PRÉPARATION D'UN CONCENTRÉ OU D'UNE POUDRE DE GINSENG FERMENTÉ

(43) Date of publication of application: 28.03.2012
(73) Proprietor: Il Hwa Co., Ltd., Gyeonggi-do 471-711 (KR)
(72) Inventor: SIM, Dae Keun, Seongnam-si Gyeonggi-do 463-765 (KR); SUNG, Jong Hwan, Namyangju-si Gyeonggi-do 472-918 (KR); LEE, Seung Kwon, Namyangju-si Gyeonggi-do 472-060 (KR); CHO, Min Goo, Guri-si Gyeonggi-do 471-745 (KR)
(74) Representative: Laufhütte, Dieter
(86) International application number: PCT/KR2009/002635
(87) International publication number: WO 2010/134650

(56) References cited:
- WO-A1-2009/057836
- FR-A1- 2 443 266
- KR-A- 19980 066 516
- KR-A- 20020 009 756
- KR-A- 20020 009 756
- KR-A- 20030 037 005
- KR-A- 20030 094 757
- US-A1- 2006 160 752
- DO YEON KIM: "Fermented ginseng attenuates hepatic lipid accumulation and hyperglycemia through AMPK activation", FOOD SCIENCE AND BIOTECHNOLOGY, vol. 18, no. 1, 1 January 2009 (2009-01-01), pages 172-178, XP055176722,

## Description

### Technical Field

The present invention relates to methods for preparing a fermented ginseng concentrate or powder.

### Background of the Invention

*Panaxginseng* C.A. Meyer (Araliaceae), is one of the medicinal plants that have long been used for treading various diseases in Asian countries, including China, Korea, and Japan. In particular, ginsenosides (ginseng saponin), the main active ingredient of *Panaxginseng*, are known to have various physiological activities, such as anti-aging activity, anti-inflammatory activity, antioxidant activities in the central nervous system, cardiovascular system, and the immune system (Wu et al., J. Immunol., 148:1519-25.1992; Lee, Facts about Ginseng, the Elixir of Life., Hollyn International. New Jersey, 1992; Huang, The Pharmacology of Chinese Herb. CRC Press. Florida, 1999), anti-diabetic activity (Chang. Pharmacology and Application of Chinese Material Medica. Vol.1, World Scientific. Singapore, 1986), and anti-tumor activity (Sato et al., Biol. Pharm. Bull. 17:635-9, 1994: Mochizuki et al., Biol. Pharm, Bull. 18:1197-1202, 1995).

Further, ginsenosides are metabolized by bacteria inside the human intestine after intake, where their metabolites are known to have various physiological activities (Karikura et al., Chem. Pharm. Bull., 39:2357-61, 1991; Kanaoda et al., J. Tradit. Med. 11:241-5, 1994; Akao et al., Biol. Pharm. Bull., 21:245-9, 1998). For example, Rb1, Rb2, and Rc, which are protopanaxadiol-type ginsenosides, are metabolized by human intestinal bacteria into IH-901 (20-O-β-D-glucopyranosyl-20(S)-protopandaxadiol) (Hasegawa et al., Planta Medica 63:463-40, 1997; Tawab et al., DrugMetab. Dispos., 31:1065-71, 2003), while Re and Rg1, protopanaxatriol-type ginsenosides, are metabolized into ginsenoside Rh1 or ginsenoside F1 (Hasegawa et al., Planta Medica 63:463-40, 1997; Tawab et al., DrugMetab. Dispos., 31:1065-, 2003), where the metabolites IH-901, Rh1, and F1 exhibit various physiological activities.

Specifically, IH-901 is generally known for its anti-diabetic (Choi et al., J. Ginseng Res. 31(2): 79-85, 2007) and immune-enhancing activities. Further, IH-901 is known to induce an anti-metastasis or anti-cancer effect by blocking tumor invasion or preventing chromosomal mutation and tumor formation (Wakabayashi et al., Oncol. Res., 9:411-7, 1998; Lee et al., Cancer Lett., 144:39-43,1999).

Several researchers have tried to produce genuine prosapogenin or sapogenin by using methods, such as chemical synthesis, subacid hydrolysis, and alkali digestion (Han et al., Planta Medica 44:146-9, 1982; Chen et al., Chem. Pharm. Bull. 35:1653-5, 1987; Elyakov et al., Synthesis of the Ginseng Glycosides and Their Analogs. Proc. 6 thInt. GinsengSymp. Seoul 74-83, 1993). However, the above methods are known to cause various side reactions, such as epimerization, hydration, and hydroxylation.

Thus, a number of methods that convert ginsenosides under mild conditions by utilizing enzymes (Ko et al., Biosci. Biotechnol. Biochem. 64:2739-43, 2000; Ko et al., PlantaMed. 69:285-6, 2003) or intestinal bacteria (Hasegawa et al., Planta Medica 63:463-40, 1997; Bae et al., J. Microbial. Biotechnol. 13:9-14, 2003) have been studied. Specifically, methods for preparing IH-901 by hydrolyzing diol-type ginsenosides with naringinase, as well as methods for preparing IH-901 by administrating the diol-type ginsenosides orally to a rat and then having them digested in the colon have been developed. However, the above preparation methods were found to have certain drawbacks in that the production yield of IH-901 is extremely low and various secondary metabolites are produced, making it difficult to obtain highly pure IH-901 (Karikura et al., Chem. Pharm. Bull. 38:2859, 1990).

More recently, other methods for preparing IH-901 by reacting a variety of enzymes with ginsenosides (Korean Laid-open Patent Publication No. 2003-94757; Korean Patent Nos. 418604 and 377546) have been studied, but the amount of IH-901 produced using these methods was found to be too low to have any significant effect. Further, the above methods involve complicated steps and are unsuitable for preparing a fermented ginseng concentrate having a high content of IH-901.

The present invention is directed to overcoming these deficiencies in the art.

Further relevant state of the art is disclosed in the following documents: KR 2002-0009756 A discloses a fermented ginseng extract obtained by reacting beta-galactosidase with a ginseng extract at 10-50°C. Do Yeon Kirn: "Fermented ginseng attenuates hepatic lipid accumulation and hyperglycemia through AMPK activation", Food science and biotechnology, vol. 18, no. 1, 1 January 2009, pages 172-178, discloses a fermented ginseng that ameliorates hyperglycemia and hyperlipidemia and that could be used as a health functional food or therapeutic agent for type 2 diabetic patients; and a method of preparation this fermented ginseng comprising adding 1,5% of beta-galactosidase (based on the total amount of the ginseng extract) and ginseng radix ethanol extract to distilled water and incubated at 50°C for 72 hr. US 2006/160752 A1 discloses the addition of pectinase to a ginseng extract (red ginseng, white ginseng and the root hair and leaf of ginseng) at 38°G for 48 hours and the concentration of the reaction mixture. FR 2 443 266 A1 discloses a method to produce a fermented ginseng extract comprising adding pectinase to ginseng and extraction with a solvent. WO 2009/057836 A1 discloses a fermented ginseng extract obtained by reacting a ginseng, red ginseng, white ginseng, fresh ginseng, ginseng tails, ginseng leaves or ginseng berries extract with beta-galactosidase.

### Summary of the Invention

The present invention relates to a method for preparing a fermented ginseng concentrate. The method first involves subjecting ginseng to an extraction with a solvent to obtain a ginseng extract. Next, pectinase and beta-galactosidase are added to the ginseng extract under conditions effective to ferment the ginseng extract. Then, the fermented ginseng extract is concentrated to produce a fermented ginseng concentrate.

The present invention also relates to a method for preparing a fermented ginseng concentrate. The method first involves suspending ginseng in a first solvent to obtain a ginseng solution. Next, pectinase and beta-galactosidase are added to the ginseng solution under conditions effective to ferment the ginseng solution. Then, the fermented ginseng solution is subjected to an extraction with a second solvent to obtain a fermented ginseng extract. Finally, the fermented ginseng extract is concentrated to produce a fermented ginseng concentrate.

The methods for preparing a fermented ginseng concentrate and fermented ginseng powder according to the present invention involve simple steps and are capable of producing a fermented ginseng concentrate or powder having a high IH-901 content. Due to their high IH-901 content, the fermented ginseng concentrate or powder prepared according to the methods of the present invention can be effectively used in functional food compositions.

### Brief Description of the Drawings

Fig. 1 is a graph depicting the results from a high-pressure liquid chromatography analysis of the fermented ginseng concentrate of the present invention.
Fig. 2 is a graph showing the average plasma concentrations of IH-901 over time for the subjects who took the fermented ginseng concentrate of the present invention.
Fig. 3 is a graph showing the maximum plasma concentrations (Cₘₐₓ) of IH-901 of each subject.
Fig. 4 is a graph showing the times to reach maximum plasma concentration (Tₘₐₓ) of IH-901 of each subject.
Fig. 5 is a graph showing the area under the plasma concentration time curve (AUC) of each subject.

### Detailed Description of the Invention

The present invention relates to a method for preparing a fermented ginseng concentrate.

The method first involves subjecting ginseng to an extraction with a solvent to obtain a ginseng extract. Examples of ginseng may include, but are not limited to, ginseng tail, white ginseng, fresh ginseng, dried ginseng, red ginseng, taekuk ginseng, Korean ginseng, Chinese ginseng, Japanese ginseng, Asian ginseng, American ginseng, extracts thereof, powders thereof, and mixtures thereof.

The solvent used in the extraction of ginseng is selected from water, ethanol and a mixture thereof.

In some embodiments, the ginseng may be subjected to an extraction with the above solvents by using any commonly known solvent extraction methods. In certain embodiments, an appropriate mixture of water and ethanol may be used for the extraction, where the ethanol in the mixture may increase the solubility of the ingredients in ginseng that are insoluble in water and/or the intermediates produced form the enzymatic reactions, resulting in an overall increase in the IH-901 content in the final product, i.e. the fermented ginseng concentrate or powder.

Next, pectinase and beta-galactosidase are added to the ginseng extract under conditions effective to ferment the ginseng extract. In some embodiments, the ginseng extract may be diluted with a solvent before the pectinase and the beta-galactosidase are added. In some embodiments, the pectinase and the beta-galactosidase are added to the ginseng extract together or at the same time. In other embodiments, the pectinase and the beta-galactosidase are added to the ginseng extract separately or at different times.

In some embodiments, the pectinase and the beta-galactosidase may be added to the ginseng extract in a relative ratio ranging from about 100:1 to about 1:100, from about 50:1 to about 1:50, from about 20:1 to about 1:20, from about 10:1 to about 1:10, from about 5:1 to about 1:5, from about 3:1 to about 1:3, or from about 2:1 to about 1:2. In other embodiments, the pectinase and the beta-galactosidase may be added to the ginseng extract in a relative ratio of about 100:1, about 50:1, about 20:1, about 10:1, about 5:1, about 3:1, about 2:1, about 1:2, about 1:3, about 1:5, about 1:10, about 1:20, about 1:50, or about 1:100.

In some embodiments, the total amount of the pectinase and beta-galactosidase added to the ginseng extract may range from about 0.01 wt.% to about 50 wt.%, from about 0.05 wt.% to about 50 wt.%, from about 0.1 wt.% to about 50 wt.%, from about 0.5 wt.% to about 50 wt.%, from about 1 wt.% to about 50 wt.%, from about 2 wt.% to about 50 wt.%, from about 5 wt.% to about 50 wt.%, from about 10 wt.% to about 50 wt.%, from about 25 wt.% to about 50 wt.%, from about 0.01 wt.% to about 0.05 wt.%, from about 0.01 wt.% to about 0.1 wt.%, from about 0.01 wt.% to about 0.5 wt.%, from about 0.01 wt.% to about 1 wt.%, from about 0.01 wt.% to about 2 wt.%, from about 0.01 wt.% to about 5 wt.%, from about 0.01 wt.% to about 10 wt.%, from about 0.01 wt.% to about 25 wt.%, from about 0.05 wt.% to about 0.1 wt.%, from about 0.1 wt.% to about 0.5 wt.%, from about 0.5 wt.% to about 1 wt.% from about 1 wt.% to about 2 wt.%, from about 2 wt.% to about 5 wt.%, from about 5 wt.% to about 10 wt.%, or from about 10 wt.% to about 25 wt.%, based on the total weight of the ginseng extract or ginseng. In other embodiments, the total amount of the pectinase and beta-galactosidase added to the ginseng extract may be about 0.01 wt.%, about 0.05 wt.%, about 0.1 wt.%, about 0.5 wt.%, about 1 wt.%, about 2 wt.%, about 5 wt.%, about 10 wt.%, about 25 wt.%, or about 50 wt.%, based on the total weight of the ginseng extract or ginseng.

In some embodiments, the pectinase and the beta-galactosidase are added to the ginseng extract where the reaction is carried out at a pH of from about 3 to about 8, from about 4 to about 8, from about 4.5 to about 8, from about 5 to about 8, from about 6 to about 8, from about 3 to about 4, from about 3 to about 4.5, from about 3 to about 5, from about 3 to about 6, from about 4 to about 4.5, from about 4.5 to about 5, or from about 5 to about 6. In other embodiments, the reaction is carried out at a pH of about 3, about 4, about 4.5, about 5, about 6, or about 8. The reaction, however, may be carried out at any pH as song as the activities of the pectinase and the beta-galactosidase are maintained. The pH may be adjusted by adding to the ginseng extract solutions, such as but not limited to, a phosphate solution, citric acid solution, and sodium citrate buffer solution.

The reaction temperature for adding the pectinase and the beta-galactosidase to the ginseng extract may be any temperature as long as the activities of the pectinase and beta-galactosidase are maintained. In some embodiments, the reaction temperature range may range from about 10°C to about 70°C, from about 30°C to about 70°C, from about 40°C to about 70°C, from about 50°C to about 70°C, from about 60°C to about 70°C, from about 10°C to about 30°C, from about 10°C to about 40°C, from about 10°C to about 50°C, from about 10°C to about 60°C, from about 30°C to about 40°C, from about 40°C to about 50°C, or from about 50°C to about 60°C. In other embodiments, the reaction temperature may be about 10°C, about 30°C, about 40°C, about 50°C, about 60°C, or about 70°C.

Further, the reaction time may be any length of time, as long as the activities of the pectinase and beta-galactosidase are maintained. In some embodiments, the reaction time may range from about 1 hour to about 72 hours, from about 12 hours to about 72 hours, from about 24 hours to about 72 hours, from about 48 hours to about 72 hours, from about 60 hours to about 72 hours, from about 1 hour to about 12 hours, from about 1 hour to about 24 hours, from about 1 hour to about 48 hours, from about 1 hour to about 60 hours, from about 12 hours to about 24 hours, from about 24 to about 48 hours, or from about 48 to about 60 hours. In other embodiments, the reaction time may be about 1 hour, about 12 hours, about 24 hours, about 48 hours, about 60 hours, or about 72 hours.

Finally, the fermented ginseng extract is concentrated to produce a fermented ginseng concentrate. In some embodiments, the concentrating of the fermented ginseng extract may involve initially carrying out a centrifugation of the fermented ginseng extract and then concentrating only the separated supernatant. In other embodiments, the concentrating of the fermented ginseng extract may involve concentrating the fermented ginseng extract without any pre-processing steps. The fermented ginseng extract may be concentrated by using commonly used devices, such as but not limited to, a vacuum decompression concentrator.

In some embodiments, the method of the present invention may further include drying the prepared fermented ginseng concentrate (which may be in a liquid form) to produce fermented ginseng powder. The drying may be carried out by any conventional drying method, such as but not limited to, drying under reduced pressure, hot-air drying, spray drying, fluidized bed drying, fluidized bed granulation, and freeze drying or lyophilization.

The present invention also relates to a method for preparing a fermented ginseng concentrate. The method first involves suspending ginseng in a first solvent to obtain a ginseng solution. Next, pectinase and beta-galactosidase are added to the ginseng solution under conditions effective to ferment the ginseng solution. Then, the fermented ginseng solution is subjected to an extraction with a second solvent to obtain a fermented ginseng extract. In some embodiments, the first and second solvents may be an aqueous solvent (e.g., water), an organic solvents, or a mixture thereof. Finally, the fermented ginseng extract is concentrated to produce a fermented ginseng concentrate. Descriptions regarding the various conditions used in this method, for example, with respect to addition of the pectinase and beta-galactosidase, extraction, and concentration already described above are not necessarily repeated herein.

### Examples

The following examples are provided to illustrate embodiments of the present invention but are by no means intended to limit its scope.

### Example 1 - Preparation of Fermented Ginseng Concentrate Using Pectinase and beta-Galactosidase

### <1-1> Preparation of Fermented Giaseng Concentrate Using Ginseng Tailand White Ginseng

5 g of ginseng tail and 5 g of white ginseng were extracted with a mixture of water and ethanol (mixing ratio of 1:1; in an amount corresponding to 500% compared to the raw materials) four times at 80°C at 3-hour intervals to obtain 7 g of ginseng extract. 100 ml of water and then 0.3 g of pectinase and 1 g of beta-galactosidase were added to the ginseng extract, where the reaction mixture was maintained at 50°C, pH 4-5, for 48 hours, The reactant was filtered and concentrated under reduced pressure at 80°C to give 6.3 g of fermented ginseng concentrate according to the present invention.

### <1-2> Preparation of Fermented Ginseng Concentrate Using Ginseng Tailand White Ginseng

7 g of ginseng extract was prepared as described in Example < 1-1> above. 100 ml of water and then 1 g of beta-galactosidase were added to the ginseng extract, where the reaction mixture was maintained at 50°C for 24 hours, and then the enzyme was inactivated by subjecting the mixture at a temperature of 80°C for 3 hours. The reactant was cooled and the pH was adjusted to 4-5 with citric acid. Then, 0.3 g of pectinase was added thereto where the mixture was subjected to a reaction for 24 hours. Subsequently, the reaction mixture was filtered and concentrated under reduced pressure at 80°C to give 5.9 g of fermented ginseng concentrate according to the present invention.

### <1-3> Preparation of Fermented Ginseng Concentrate Using Ginseng Tailand White Ginseng

5 g of ginseng tail and 5 g of white ginseng were extracted with a mixture of water and ethanol (mixing ratio of 1:1; in an amount corresponding to 500% compared to the raw materials) to obtain 7 g of ginseng extract. 100 ml of water and then 0.5 g of pectinase and 0.7 g of beta-galactosidase were added to the ginseng extract, where the reaction mixture was maintained at 50°C, pH 4-5, for 48 hours. The reactant was filtered and concentrated under reduced pressure at 80°C to give 6.2 g of fermented ginseng concentrate according to the present invention.

### <1-4> Preparation of Fermented Ginseng Concentrate Using Ginseng Tailand White Ginseng

5 g of ginseng tail and 5 g of white ginseng were extracted with a mixture of water and ethanol (mixing ratio of 1:1; in an amount corresponding to 500% compared to the raw materials) to obtain 7 g of ginseng extract. 100 ml of water and then 0.9 g of pectinase and 0.6 g of beta-galactosidase were added to the ginseng extract, where the reaction mixture was maintained at 50°C. pH 4-5, for 48 hours. The reactant was filtered and concentrated under reduced pressure at 80°C to give 5.9 g of fermented ginseng concentrate according to the present invention.

### <1-5> Preparation of Fermented Ginseng Concentrate Using Ginseng Tailand White Ginseng

5 g of ginseng tail and 5 g of white ginseng were extracted with a mixture of water and ethanol (mixing ratio of 1:1; in an amount corresponding to 500% compared to the raw materials) to obtain 7 g of ginseng extract, 100 ml of water and then 1.2 g of pectinase and 0.2 g of beta-galactosidase were added to the ginseng extract, where the reaction mixture was maintained at 50°C, pH 4-5, for 48 hours. The reactant was filtered and concentrated under reduced pressure at 80°C to give 6.3 g of fermented ginseng concentrate according to the present invention.

### <1-6> Preparation of Fermented Ginseng Concentrate Using Ginseng Tail

10 g of ginseng tail was extracted with 100 ml of water to obtain 7 g of ginseng extract. The pH of the extract was adjusted to 3-4 with citric acid. 0.3 g of pectinase and 1 g of beta-galactosidase were added to the ginseng extract, where the mixture was subjected to a reaction at 50°C for 48 hours. Subsequently, the reactant was filtered and concentrated by the same method as described in Example <1-1> above to obtain 6.5 g of fermented ginseng concentrate (I) according to the present invention.

Alternatively, 10 g of ginseng tail was extracted with a mixture of water and ethanol by the same method as described in Example <1-1> above to obtain 7.2 g of ginseng extract. The pH of the extract was adjusted to 3-4 with citric acid. 0.3 g of pectinase and 1 g of beta-galactosidase were added to the ginseng extract, where the mixture was filtered and concentrated by the same method as described in Example <1-1> above to obtain 6.8 g of fermented ginseng concentrate (II) according to the present invention.

### <1-7> Preparation of Fermented Ginseng Concentrate Using Ginseng Tail

10 g of ginseng tail was extracted with 100 ml of water to obtain 7 g of ginseng extract. 100 ml of water and then 1 g of beta-galactosidase were added to the ginseng extract where the reaction mixture was maintained at 50°C for 24 hours, and then the enzyme was inactivated by subjecting the mixture at a temperature of 80°C for 3 hours. The reactant was cooled and the pH was adjusted to 4-5 with citric acid. Then, 0.3 g of pectinase was added thereto and reacted at 50°C for 24 hours. Subsequently, the reactant was filtered and concentrated under reduced pressure at 80°C to obtain 6.5 g of fermented ginseng concentrate according to the present invention.

### <1-8> Preparation of Fermented Ginseng Concentrate Using White Ginseng

1 kg of white ginseng was extracted with 5ℓ of ethanol to obtain 540 g of ginseng extract. 7 g of the extract was concentrated and then suspended in water. 0.3 g of pectinase and 1 g of beta-galactosidase were added thereto, where the mixture was reacted, filtered, and concentrated by the same method as described in Example <1-1> above to obtain 5.2 g of fermented ginseng concentrate according to the present invention.

### <1-9> Preparation of Fermented Ginseng Concentrate Using White Ginseng

1 kg of white ginseng was extracted with 5ℓ of ethanol to obtain 540 g of ginseng extract. 7 g of the extract was concentrated and then suspended in water. 0.5 g of pectinase and 0.5 g of beta-galactosidase were added thereto, where the mixture was reacted, filtered, and concentrated by the same method as described in Example <1-1> above to obtain 5.1 g of fermented ginseng concentrate according to the present invention.

### <1-10> Preparation of Fermented Ginseng Concentrate Using White Ginseng

1 kg of white ginseng was extracted with 5ℓ of ethanol to obtain 540 g of ginseng extract. 7 g of the extract was concentrated and then suspended in water. 1.5 g of pectinase and 0.2 g of beta-galactosidase were added thereto, where the mixture was reacted, filtered, and concentrated by the same method as described in Example <1-1> above to obtain 6.2 g of fermented ginseng concentrate according to the present invention.

### <1-11> Preparation of Fermented Ginseng Concentrate Using Fresh Ginseng

6 g of fresh ginseng was sliced into pieces and sterilized. After the ginseng pieces were suspended in water, the pH was adjusted to 3-4.5 with citric acid, and 0.3 g of pectinase and 1 g of beta-galactosidase were added thereto where the mixture was subjected to a reaction at 50°C for 72 hours. The reactant was extracted with a mixture of water and ethanol prepared by the same method as described in Example <1-1> above four times at 80°C for 4 hours. Then, the extract was filtered and concentrated to obtain 3.5 g of fermented ginseng concentrate according to the present invention.

### <1-12> Preparation of Fermented Ginseng Concentrate Using Fresh Ginseng

6 g of fresh ginseng was sliced into pieces and sterilized. After the ginseng pieces were suspended in water, 1g of beta-galactosidase was added thereto where the mixture was subjected to a reaction at 50°C for 24 hours and inactivated at 80°C for 3 hours. The reactant was cooled and the pH was adjusted to 4-5 with citric acid. Then, 0.3 g of pectinase was added thereto and reacted at 50°C for 24 hours. Subsequently, the reactant was filtered and then extracted with a mixture of water and ethanol prepared by the same method as described in Example <1-1> above four times at 80°C for 4 hours. Then, the extract was concentrated under reduced pressure at 80°C to obtain 2.9 g of fermented ginseng concentrate according to the present invention.

### <1-13> Preparation of Fermented Ginseng Concentrate Using Fresh Ginseng

6 g of fresh ginseng was sliced into pieces and sterilized. After the ginseng pieces were suspended in water, the pH was adjusted to 3-4 with citric acid. Then, 0.7g of pectinase and 0.6g of beta-galactosidase were added thereto where the mixture was subjected to a reaction at 50°C for 72 hours. Subsequently, the reactant was filtered and concentrated to obtain 2.9 g of fermented ginseng concentrate according to the present invention.

### <1-14> Preparation of Fermented Ginseng Concentrate Using Fresh Ginseng

6 g of fresh ginseng was sliced into pieces and sterilized. After the ginseng pieces were suspended in water, 0.7g of beta-galactosidase was added thereto where the mixture was subjected to a reaction at 50°C for 24 hours and inactivated at 80°C for 3 hours. The reactant was cooled and the pH was adjusted to 4-5 with citric acid. Then, 0.6 g of pectinase was added thereto and reacted at 50°C for 24 hours. Subsequently, the reactant was filtered and then extracted with a mixture of water and ethanol prepared by the same method as described in Example <1-1> above four times at 80°C. for 4 hours. Then, the extract was concentrated under reduced pressure at 80°C to obtain 2.3 g of fermented ginseng concentrate according to the present invention.

### <1-15> Preparation of Fermented Ginseng Concentrate Using Fresh Ginseng

6 g of fresh ginseng was sliced into pieces and sterilized. After the ginseng pieces were suspended in water, the pH was adjusted to 3-4.5 with citric acid. Then, 0.9g of pectinase and 0.5g of beta-galactosidase were added thereto where the mixture was subjected to a reaction at 50°C for 72 hours. Subsequently, the reactant was filtered and concentrated to obtain 2.5 g of fermented ginseng concentrate according to the present invention.

### <1-16> Preparation of Fermented Ginseng Concentrate Using Fresh Ginseng

6 g of fresh ginseng was sliced into pieces and sterilized. After the ginseng pieces were suspended in water, the pH was adjusted to 3-4.5 with citric acid. Then, 2 g of pectinase and 0.2 g of beta-galactosidase were added thereto where the mixture was subjected to a reaction at 50°C for 72 hours. Subsequently, the reactant was filtered and concentrated to obtain 3.3 g of fermented ginseng concentrate according to the present invention.

### <1-17> Preparation of Fermented Ginseng Concentrate Using Fresh Ginseng

6 g of fresh ginseng was sliced into pieces and sterilized. After the ginseng pieces were suspended in water, 0.5 g of beta-galactosidase was added thereto where the mixture was subjected to a reaction at 50°C for 24 hours and inactivated at 80°C for 3 hours. The reactant was cooled and the pH was adjusted to 4-5 with citric acid. Then, 1.5 g of pectinase was added thereto and reacted at 50°C for 24 hours. Subsequently, the reactant was filtered and then extracted with a mixture of water and ethanol prepared by the same method as described in Example <1-1> above four times at 80°C for 4 hours. Then, the extract was concentrated under reduced pressure at 80°C to obtain 3.1 g of fermented ginseng concentrate according to the present invention.

### <1-18> Preparation of Fermented Ginseng Concentrate Using Ginseng Powder

(102) 7 g of ginseng powder was suspended in water. The pH was adjusted to 3-4.5 with nitric acid. The suspended ginseng powder was reacted with 0.3 g of pectinase and 1 g of beta-galactosidase and extracted by the same method as described in Example <1-11> above, and then filtered and concentrated to obtain 3.7 g of fermented ginseng concentrate according to the present invention.

### Comparative Example 1 - Preparation of Fermented Ginseng Concentrate Using PectinaseOnly

100 ml of water was added to 7 g of ginseng extract prepared by the same method as described in Example <1-1> above. Then, 1.3 g of pectinase was added to the extract, which was subsequently reacted and concentrated under reduced pressure by the same method as described in Example <1-1> above to obtain 5.6 g of fermented ginseng concentrate according to the present invention.

### Comparative Example 2 - Preparation of Fermented Ginseng Concentrate Using beta-Galactosidase Only

100 ml of water was added to 7 g of ginseng extract prepared by the same method as described in Example <1-1> above. Then, 1.3 g of beta-galactosidase was added to the extract, which was subsequently reacted and concentrated under reduced pressure by the same method as described in Example <1-1> above to obtain 5.4 g of fermented ginseng concentrate according to the present invention.

### Experimental Example 1 - TheIH-901 Content in the Fermented Ginseng Concentrate of the present Invention

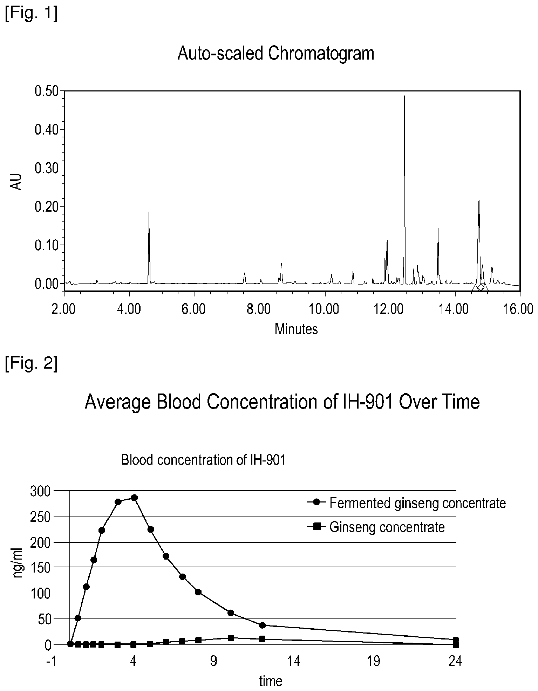

In order to analyze the content of IH-901 (20-O-β-D-glucopyranosyl-20(S)-protopanaxadiol) in the fermented ginseng concentrate prepared by the method described in Example <1-1> above, 1 g of the obtained fermented ginseng concentrate was taken and extracted with 60 ml of water-saturated butanol four times. The butanol fraction was concentrated to obtain 120 mg of concentrate, and the concentrate was dissolved in methanol to analyze the IH-901 content by ultra performance liquid chromatography (UPLC). The results of the UPLC analysis are shown in Fig. 1. The IH-901 content in the ginseng extract prepared in Example <1-1> above was also measured in the same manner as above.

Further, the IH-901 content in the fermented ginseng concentrate prepared by the method described in Comparative Example 1 or Comparative Example 2 above was analyzed in the same manner as above.

The results of the above analyses are summarized in Table 1 below.

### Table 1

**[Table 1]**

| [Table ] | | | | |
|---|---|---|---|---|
| Component | Content of each component | | | |
| | Ginseng Extract of Example 1-1 | Fermented Ginseng Concentrate of Example 1-1 | Fermented Ginseng Concentrate of Comp. Example 1 | Fermented Ginseng Concentrate of Comp. Example 2 |
| Ginsenoside Rb1 | 17,6 mg/g | 1.2 mg/g | 6.3 mg/g | 3.2 mg/g |
| Ginsenoside Rb2 | 14.6 mg/g | 1.1 mg/g | 7.1 mg/g | 5.7 mg/g |
| Ginsenoside Rc | 18.9 mg/g | 0.1 mg/g | 9.3 mg/g | 6.3 mg/g |
| Ginsenoside Rd | 7.9 mg/g | 1.4 mg/g | 2.2 mg/g | 4.2 mg/g |
| IH-901 | 0 mg/g | 36 mg/g | 24 mg/g | 10 mg/g |

As shown in Table 1 above, the fermented ginseng concentrate according to the present invention, in contrast to the ginseng extract, contains a large amount of IH-901. Furthermore, the IH-901 content in the fermented ginseng concentrate according to the present invention is 3.6 times higher than that in the fermented ginseng concentrate prepared using beta-galactosidase only and 1.5 times higher than that in the fermented ginseng concentrate prepared using pectinase only.

### Experimental Example 2 - Study on Body's Absorption of IH-901 of the Fermented Ginseng Concentrate of the Present Invention

Twenty four healthy male volunteers of ages ranging from 20 to 45, whose body weights are within 20% of the ideal body weights, were used as subjects for the study (randomized, 2 X 2 cross-over design).

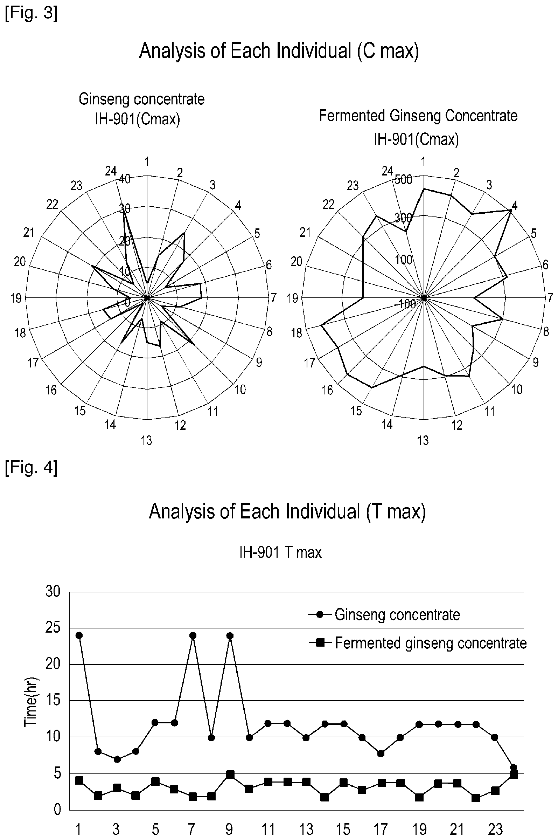

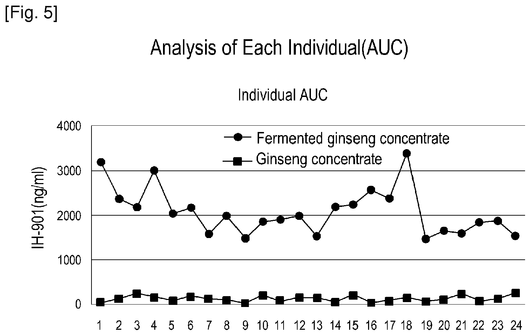

Each volunteer received the fermented ginseng extract or ginseng extract for pharmacokinetic characteristic assessments. Blood samples were taken at 0, 0.5, 1, 1.5, 2, 3, 4, 5, 6, 7, 8, 10, 12, and 24 hours after administration of the fermented ginseng extract or ginseng extract and were analyzed by LC-MS/MS. The average plasma concentrations of IH-901 over time are shown in Fig. 2. The average maximum plasma concentration (Cₘₐₓ), average time to reach maximum plasma concentration (Tₘₐₓ), and area under the plasma concentration time curve (AUC) values measured are shown in Table 2 below, where Cₘₐₓ is the average of the maximum concentration values of each subject irrespective of time and Tₘₐₓ is the average of the time values corresponding to the Cₘₐₓ values of each subject. The Cₘₐₓ, Tₘₐₓ, and AUC values for each subject are shown in Figs. 3 to 5.

### Table 2

**[Table 2]**

| [Table ] | | | |
|---|---|---|---|
| Ð | Cₘₐₓ Ð | Tₘₐₓ Ð | AUC |
| Fermented ginseng concentrate | 325.00±91.97 ng/ml | 3.29 hr | 2083.09±524.68 ng/ml |
| Ginseng concentrate | 13.88±7.24 ng/ml | 12.04 hr | 134.5±63.10 ng/ml |

As shown in Table 2 above, the Cₘₐₓ, AUC, and Tₘₐₓ of IH-901 in the fermented ginseng concentrate are about 24 times higher, 15 times higher, and 3.7 times lower than those of the ginseng concentrate, respectively.

Therefore, it was found that when a ginseng concentrate was taken, each individual had different capabilities with respect to metabolizing ginsenosides to IH-901 and only a small amount of IH-901 was absorbed in the body. Further, when a ginseng concentrate was taken, each individual exhibited different average times in reaching the maximum plasma concentration.

In contrast, when a fermented ginseng concentrate was taken, the amount of IH-901 absorbed and the average time to reach maximum plasma level were constant for each individual. Further, when a fermented ginseng concentrate was taken, a large amount of IH-901 was absorbed in the body.

Accordingly, the above results demonstrate that a steady and constant effect (with little individual variation) can be expected when subjects take fermented ginseng concentrate.

### Example 2 - Preparation of Fermented Ginseng Powder According to the Present Invention

### <2-1> Preparation of Fermented Ginseng Powder Using Ginseng Tailand White Ginseng

100 g of water was added to 7 g of an extract of ginseng prepared by the same method as described in Example <1-1> above. Then, 0.3 g of pectinase and 1 g of beta-galactosidase were added to the extract, which was subsequently reacted at 50°C, pH 4-5, for 24 hours. The reacted extract was dried under reduced pressure at 80°C to obtain 4.23 g of fermented ginseng powder according to the present invention.

### <2-2> Preparation of Fermented Ginseng Powder Using Ginseng Powder

7 g of ginseng powder was suspended in water, and then the pH was adjusted to 3-4.5. Next, 0.3 g of pectinase and 1 g of beta-galactosidase were added to the suspension, which was subsequently reacted at 50°C for 24 hours and extracted with a mixture of water and ethanol prepared by the same method as in described in Example <1-1> above four times at 80°C for 4 hours. The extract was then dried under reduced pressure to obtain 2.6 g of fermented ginseng powder.

## Claims

1. A method for preparing a fermented ginseng concentrate comprising:
subjecting ginseng to an extraction with a solvent selected from water, ethanol, and a mixture thereof to obtain a ginseng extract;
adding pectinase and beta-galactosidase to the ginseng extract under conditions effective to ferment the ginseng extract; and
concentrating the fermented ginseng extract to produce a fermented ginseng concentrate.

2. The method according to Claim 1, wherein the ginseng is one or more of ginseng tail, white ginseng, fresh ginseng, dried ginseng, red ginseng, taekuk ginseng, Korean ginseng, Chinese ginseng, Japanese ginseng, Asian ginseng, American ginseng, extracts thereof, powders thereof, and mixtures thereof.

3. The method according to Claim 1, wherein said adding comprises adding the pectinase and the beta-galactosidase together.

4. The method according to Claim 1, wherein said adding comprises adding the pectinase and the beta-galactosidase separately.

5. The method according to Claim 1, wherein said adding comprises adding the pectinase and the beta-galactosidase in a relative ratio ranging from 100:1 to 1:100, wherein the amount of the pectinase and beta-galactosidase ranges from 0.01 wt% to 50 wt% based on the total amount of the ginseng extract or ginseng.

6. The method according to Claim 1, wherein said adding comprises adding the pectinase and the beta-galactosidase to the ginseng extract at a temperature of from 10°C to 70°C.

7. The method according to Claim 1, wherein said adding comprises adding the pectinase and the beta-galactosidase to the ginseng extract for a time period from 1 to 72 hours.

8. The method according to Claim 1 further comprising:
drying the fermented ginseng concentrate after said concentrating to produce fermented ginseng powder.

9. The method according to Claim 8, wherein said drying is carried out by drying under reduced pressure, hot-air drying, spray drying, fluidized bed drying, fluidized bed granulation, or freeze drying.

## Patentansprüche

1. Verfahren zum Erzeugen eines fermentierten Ginsengkonzentrats, umfassend:
Unterziehen von Ginseng einer Extraktion mit einem Lösungsmittel gewählt aus Wasser, Ethanol und einer Mischung derselben, um einen Ginsengextrakt zu erhalten;
Zugeben von Pektinase und Beta-Galactosidase zu dem Ginsengextrakt unter Bedingungen, bei denen der Ginseng fermentiert wird; und
Konzentrieren des fermentierten Ginsengextrakts, um ein fermentiertes Ginsengkonzentrat herzustellen.

2. Verfahren nach Anspruch 1, wobei es sich bei dem Ginseng um Ginsengschwanz, weißen Gingseng, frischen Ginseng, getrockneten Ginseng, roten Ginseng, Taekuk Ginseng, koreanischen Ginseng, chinesischen Ginseng, japanischen Ginseng, asiatischen Ginseng, amerikanischen Ginseng, Extrakte desselben, Pulver desselben und/oder Mischungen desselben handelt.

3. Verfahren nach Anspruch 1, wobei das Zugeben das gemeinsame Zugeben der Pektinase und der Beta-Galactosidase umfasst.

4. Verfahren nach Anspruch 1, wobei das Zugeben das getrennte Zugeben der Pektinase und der Beta-Galactosidase umfasst.

5. Verfahren nach Anspruch 1, wobei das Zugeben das Zugeben der Pektinase und der Beta-Galactosidase bei einem von 100:1 bis 1:100 reichenden relativen Verhältnis umfasst, wobei die Menge der Pektinase und der Beta-Galactosidase beruhend auf der Gesamtmenge des Ginsengextrakts oder Ginsengs von 0,01 Gew.-% bis 50 Gew.-% reicht.

6. Verfahren nach Anspruch 1, wobei das Zugeben das Zugeben der Pektinase und der Beta-Galactosidase zu dem Ginsengextrakt bei einer Temperatur von 10°C bis 70°C umfasst.

7. Verfahren nach Anspruch 1, wobei das Zugeben das Zugeben der Pektinase und der Beta-Galactosidase zu dem Ginsengextrakt über einen Zeitraum von 1 bis 72 Stunden umfasst.

8. Verfahren nach Anspruch 1, weiterhin umfassend:
Trocknen des fermentierten Ginsengkonzentrats nach dem Konzentrieren, um fermentiertes Ginsengpulver herzustellen.

9. Verfahren nach Anspruch 8, wobei das Trocknen ausgeführt wird durch Trocknen unter verringertem Druck, Heißlufttrocknen, Sprühtrocknen, Wirbelschichttrocknen, Wirbelschichtgranulation oder Gefriertrocknen.

## Revendications

1. Procédé de préparation d'un concentré de ginseng fermenté, comprenant :
la soumission de ginseng à une extraction avec un solvant choisi parmi l'eau, l'éthanol, et un mélange de ceux-ci afin d'obtenir un extrait de ginseng ;
l'ajout de pectinase et de bêta-galactosidase à l'extrait de ginseng dans des conditions efficaces pour fermenter l'extrait de ginseng ; et
la concentration de l'extrait de ginseng fermenté pour produire un concentré de ginseng fermenté.

2. Procédé selon la revendication 1, dans lequel
le ginseng est des radicelles de ginseng, du ginseng blanc, du ginseng frais, du ginseng séché, du ginseng rouge, du ginseng taekuk, du ginseng coréen, du ginseng chinois, du ginseng japonais, du ginseng asiatique, du ginseng américain, des extraits de ceux-ci, des poudres de ceux-ci, et/ou des mélanges de ceux-ci.

3. Procédé selon la revendication 1, dans lequel
ledit ajout comprend l'ajout de la pectinase et de la bêta-galactosidase ensemble.

4. Procédé selon la revendication 1, dans lequel
ledit ajout comprend l'ajout de la pectinase et de la bêta-galactosidase séparément.

5. Procédé selon la revendication 1, dans lequel
ledit ajout comprend l'ajout de la pectinase et de la bêta-galactosidase dans un rapport relatif allant de 100:1 à 1:100, la quantité de la pectinase et de la bêta-galactosidase allant de 0,01 % en poids à 50 % en poids sur la base de la quantité totale de l'extrait de ginseng ou du ginseng.

6. Procédé selon la revendication 1, dans lequel
ledit ajout comprend l'ajout de la pectinase et de la bêta-galactosidase à l'extrait de ginseng à une température comprise entre 10 °C et 70 °C.

7. Procédé selon la revendication 1, dans lequel
ledit ajout comprend l'ajout de la pectinase et de la bêta-galactosidase à l'extrait de ginseng pendant une durée comprise entre 1 et 72 heures.

8. Procédé selon la revendication 1, comprenant en outre :
le séchage du concentré de ginseng fermenté après ladite concentration pour produire de la poudre de ginseng fermenté.

9. Procédé selon la revendication 8, dans lequel
ledit séchage est exécuté par séchage sous pression réduite, séchage à air chaud, séchage par atomisation, séchage à lit fluidisé, granulation à lit fluidisé, ou lyophilisation.
